# EUROPEAN PATENT APPLICATION

(11) **EP 4 691 519 A1**
(43) Date of publication of application: **11.02.2026**
(21) Application number: 24193922.2
(22) Date of filing: 09.08.2024
(51) Int. Cl.: A61M 5/20, A61M 5/32, A61M 5/24

(54) **RADIATION TRANSMISSION IN AN INJECTION DEVICE**

(71) Applicant: Ypsomed AG, 3401 Burgdorf (CH)
(72) Inventor: Scheurer, Simon, 3006 Bern (CH); Hostettler, Patrick, 3415 Hasle (CH); Klötzli, Urs, 3414 Oberburg (CH); Kühni, Florian, 3007 Bern (CH)

(57) **Abstract**

The invention relates to an injection device assembly for dispensing a liquid drug from a reservoir (6) through an injection needle (5). The injection device assembly comprises a device housing (20), a radiation emitter (21) arranged in a distal end portion of the device housing (20) and configured to emit electromagnetic radiation (22) and a cap (10) releasably attachable to a distal end of the injection device assembly. The cap (10) is attached in a storage state or shipping state and can be removed by the user prior injection. The cap (10) comprises a translucent or transparent part with a radiation capturing surface (13) adapted to capture electromagnetic radiation (22) emitted by the emitter element (21) and with a radiation emitting surface (15) adapted to emit, based on the radiation captured, an indication to a user.

## Description

### FIELD OF THE INVENTION

The present invention relates to an injection device assembly for an injection device for injecting, delivering, administering, infusing or dispensing substances and/or liquids such as insulin, hormone preparations or vaccines. The injection device assembly comprises a device housing for accommodating a dispensing mechanism a light emitter arranged in a distal end portion of the device housing and a cap releasably attachable to a distal end of the injection device assembly.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as an electric motor or a mechanical strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

Furthermore, there are known electronic injection devices including an electronic module or units embedded or integrated in an injection device housing or being part of an auxiliary or supplemental electronic module or add-on device releasably attachable to the delivery device. Those electronic module or units may monitor a drug delivery process, in order to proactively prevent false handling of the device and/or to keep track of the doses already applied, and generates data related to an instantaneous condition and/or use of the delivery device. Several user interfaces are known for injection devices. Often are LEDs or displays implemented to indicate a current device condition or to display a requested input from a user.

EP 2549915 A1 discloses an injection device and an add-on module releasably attachable to a housing of the injection device. The add-on includes LEDs used to illuminate a dosage window in the injection device housing. In the dosing window a currently selected dose is displayed.

WO 24017715 A1 discloses an autoinjector with a rear closure cap permanently connected to a device housing. The rear closure or a part of the closure cap that is made from a transparent or translucent material. The autoinjector includes LED that is backlighting the closure cap.

WO16064916 A1 discloses an add-On for an injection pen as two-part pen sensor cap with light emitter and light sensors. The light emitter and light sensor are oriented on opposite sides of the cap. The light sensors receive a pattern of light from the light emitter that corresponds to a number of factors including the plunger position, the clarity of the insulin in the reservoir, and air bubbles in the reservoir.

### DESCRIPTION OF THE INVENTION

It is an objective of the invention to provide a signal or indication that is easy and clearly visible to the user from an injection device

This objective is achieved by the drug delivery device and the method according to the independent claims. Preferred embodiments are evident from the dependent claims.

The invention relates to an injection device assembly for dispensing a liquid drug from a reservoir through an injection needle. The injection device assembly comprises
- an injection device housing for accommodating a dispensing mechanism for dispensing the drug,
- a radiation emitter element arranged in a distal end portion of the device housing and configured to emit electromagnetic radiation,
- a cap releasably attachable to a distal end of the device housing, wherein the cap is attached in a storage state or shipping state and can be removed by the user prior injection to uncover the injection needle.
The cap comprises a translucent or transparent part fitted or adjusted to a wavelength of emitted radiation. The cap includes a radiation capturing surface adapted to capture electromagnetic radiation emitted by the emitter element. Besides the capturing surface the cap further includes a radiation emitting surface adapted to emit, based on the radiation captured, an indication to a user.

Thus, electromagnetic radiation can be transmitted from the radiation emitter element to the cap via the capturing surface (entry surface) and re-emitted (again emitted) by the translucent or transparent part of the cap when the cap is attached to the injection device assembly.

That means the radiation emitter element is configured to emit electromagnetic radiation which radiation can enter the capturing surface when the cap is attached or mounted to the injection device in the storage state or shipping state. Therefore, the electromagnetic radiation is (at least partly, preferably nearly completely) transmitted from the radiation emitter element of the housing to the cap. As the cap comprises or is made of translucent or transparent material the transmitted electromagnetic radiation is emitted to the environment by the cap and thus providing a visible indication to the user.

In an embodiment where the electromagnetic radiation is visible light the user can see the transmitted and emitted light of in the cap, for example in form of one colour or more than one distinct colours. In an alternative embodiment where the electromagnetic radiation is non-visible radiation such as ultraviolet (UV) radiation or infrared radiation the cap may include, for example, UV-fluorescent ink or UV-fluorescent printings that become visible under daylight exclusively when the cap emits the transferred UV radiation.

The radiation emitting cap allows to communicate a device status or condition to the user, for example, from an electronic unit inside the device housing. For example, a controller of the electronic unit may control the radiation emitter element to provide a signal to the user for a specific device condition or if a user action is required, for example, if the device is unlocked and the user can remove the cap (ready to use signal) or if a sensor detects a user manipulation (dose setting mechanism) on the device or if the device is in an error condition (alert signal) or is not ready to use.

The emitted radiation by the cap according to the invention provides thus a simple and easy communication from the device to the user. As the translucent or transparent part with its emitting surface serves as an output member no specific or additional and costly display or user interface is required. That simplifies the design and may reduce the overall costs of the injection device assembly and the complete injection device.

The invention relates to an injection device assembly. The term assembly means several components required to emit, transfer and re-emit the radiation by the cap are encompassed. That means the assembly must not comprise all components of a complete injection device as for the claimed radiation emittance a dose and/or dispensing mechanism is not mandatory.

The injection device may be a disposable, reusable or semi-disposable (semi-reusable) injection device. The assembly preferably includes an electronic unit or module or electronics and a controller inside the housing configured to control the emitter and preferably also a radiation source located in the same place as the emitter (same component) or alternatively spatially separated from the radiation emitter. That means the emitter may be additionally the source generating the radiation or alternatively the emitter and the source may be two distinctive components separated from each other as mentioned above. In the latter case the source may be connected to the emitter by a wire, for example, by an optic fibre cable to transfer the electromagnetic radiation from the source to the emitter.

The electromagnetic radiation comprises electromagnetic waves which may be of different frequency or wavelength as commonly known in form of the electromagnetic spectrum. The electromagnetic radiation may be classified by wavelength, for example, into infrared radiation, visible light and ultraviolet radiation (non-visible light) and further classes. The electromagnetic radiation emitted by the emitter element according to the invention is preferably visible light and/or ultraviolet (UV) radiation (which may be made visible via UV ink or printing).

The cap includes a translucent or transparent part which is made of translucent or transparent material, or it may comprise translucent or transparent material or the cap may include a translucent or transparent element. The translucent material allows radiation to pass through the material. For example, if the radiation enters the capturing surface in a proximal portion of the cap the radiation may pass through the cap and may be emitted by the emitting surface in a distal portion of the cap. The material may include scattering pigments improving the emittance of the electromagnetic radiation.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the radiation capturing surface is positioned perpendicular to an emission axis of the emitted radiation such that the emitted electromagnetic radiation can enter the capturing surface in orthogonal orientation. Preferably, the emission axis is parallel to a longitudinal axis of the emitter element.

That means the angle between the emission axis of the radiation and the capturing surface is about 90°. That allows an efficient and reliable transmission of the radiation from the emitter element in the housing to the cap such that the radiation enters the cap substantially without reflection.

The capturing surface is preferably a smooth or polished surface. All other outer surfaces apart from the capturing surface of the translucent or transparent material are preferably a rough or unpolished surface. The smooth or polished surface provides for a reliably entry of the electromagnetic radiation into the translucent or transparent material substantially without reflection. In contrast thereto, the rough or unpolished surface provides for an optimal emittance of the electromagnetic radiation to the environment. That means the radiation is emitted in all directions.

Preferably, the capturing surface is oriented to a longitudinal axis of the device housing in an angle between 30 and 60 degrees, preferably between 40 and 50 degrees. Such an orientation allows an optimal positioning of the emitter. That means the emitter element can be positioned such that an emission axis of the emitted radiation is perpendicular to the capturing surface.

Preferably, the light emitter is a Light Emitting Diode (LED). The LED is easily available, has a low energy consumption and reliably provides visible light in distinct colors. Hence, the cap can be illuminated with distinct colors by using only one LED component.

In this embodiment the LED is not only the light emitter but also a light source as the light is generated by the LED components.

In a preferred embodiment the radiation emitter is a light emitter adapted to emit visible light. That means the user can see the emitted light under daylight. The spectrum of light visible by a human eye is about 380 to about 750 nanometers.

In an alternative embodiment the radiation emitter is spatially separated from a radiation source generating the radiation. In this embodiment the radiation emitter element is preferably formed by a second end of a radiation transporting guide, for example, a fibre optic cable and a first end of the guide is optically coupled to the radiation source. That means if the radiation source is physically or spatially separated from the second end and the electromagnetic radiation is transmitted by the optic guide from its first end to its second end. The radiation source may be located in a proximal portion of the injection device assembly, for example, in an electronic unit.

Alternatively, the emitter element can the same component as the radiation source, or the emitter element is located at the same place as the radiation source. That may be the case in the above-described embodiment, if the emitter is a LED which is configured to generate visible light and to emit the light.

Moreover, the injection device assembly preferably includes a radiation sensor configured to detect electromagnetic radiation reflected or emitted by emitting surface of the cap. The sensor may be configured to generate a sensor signal based on the detection of emitted or reflected radiation and the injection device assembly may further include a controller adapted to determine based on the radiation sensor signal whether or not the cap is attached to the injection device assembly. The radiation may be visible light or alternatively non-visible radiation such as infrared or UV radiation. That means based on the detected strength or intensity or based on a presence of radiation the controller can determine if the cap is mounted to the distal end of the injection device assembly or if the cap is not present when the user has already removed the cap from the device.

The sensor is preferably located in a distal portion of the device to optimally detect radiation emitted or reflected by the emitting surface of the cap. The sensor is preferably a photoelectric sensor, for example, a semiconductor-based photodetector such as a photodiode.

The sensor is thus advantageous to determine whether the user has removed the cap. The controller may communicate the detected state to the user, for example, by a LED or display or the information may be transmitted to an external device by means of a communication module of the injection device assembly.

Preferably, the injection device assembly further includes a lock mechanism preventing or blocking a dispensing with the dispensing mechanism in a locked state. The lock mechanism allows a dispensing in an unlocked state. The locking or unlocking may be implemented by software only controlling an electric motor or alternatively the lock mechanism may comprise a physical locking member which, for example, may engage and block a dispensing member (e.g. a driver or plunger rod) in the locked state to prevent a dispensing movement and may be disengage the dispensing member in the unlocked state to allow the dispensing movement.

Preferably, the electromagnetic radiation is visible light and in the locked state a light of a first color is emitted and in the unlocked state a light of a second color distinct or different than the first color is emitted by the light emitter. The user can thus recognize in what state the injection device is. By way of example, the emitter element may emit green light if the device is ready to use and the emitter element may emit red light if the device is locked or if the device is not ready to use due to a missing release signal or due to an error or fault in the device mechanism.

In an alternative to the above-described visible light or in addition the radiation emitter element may be configured to emit UV radiation. In this embodiment the cap preferably includes UV-fluorescent ink or a UV-fluorescent printing which is not visible under daylight, but which is exclusively visible when the ink or printing is under UV radiation.

The means in this embodiment the cap may include a UV-fluorescent printing or UV-fluorescent sign which is not visible under daylight to the user. The printing or sign becomes exclusively visible when the emitter element emits UV radiation. For example, the cap may include a removal sign indicating the user that the device is ready, and the cap can be removed. The removal sign is not visible to the user in a shipping or initial state and becomes only visible when the device is ready to be used. That may facilitate the communication of device condition to the user without requiring a complex and costly user interface such as an electronic display.

Preferably, the translucent or transparent part includes scattering pigments of white color. The pigments may improve the emittance of the electromagnetic radiation, in particular the emittance of visible light such that the user can see light emitted by the emitting surface of the cap. In a preferred embodiment the amount of pigments is 10% or at least 10% of the translucent or transparent material.

In a preferred embodiment the cap includes a circumferential outer wall and an inner holding structure adapted to be connected to a rigid needle shield shielding an injection needle. The outer wall is made of the translucent or transparent part or comprises the translucent or transparent material. In this embodiment the capturing surface is arranged on a proximal end of the outer wall.

That means the ridged needle shield can be reliably removed from the injection needle along with the cap when the cap is detached and removed from the distal end of the injection device. That means in turn that upon cap removal the sterile barrier is released, and the injection needle is unshielded.

The inner holding structure is preferably an elastic tubular sleeve. The elastic sleeve can be shrunken onto the rigid needle shield during manufacturing and is thus reliably connected to rigid needle sleeve. Alternatively, the holding structure may be connected to the rigid needle in other ways, for example, by form fit only or by a frictional connection.

The invention relates further to an injection device comprising the above-described injection device assembly. The injection device is preferably a semi-reusable device comprising a disposable reservoir unit including the cap with the capturing surface and is adapted to the hold the reservoir with the liquid drug and a reusable drive unit including the device housing and the emitter element.

The reusable drive unit may include an electronic drive such as an electromotor adapted to move a plunger rod or other dispensing member in a dispensing direction to dispense the drug from the reservoir. Alternatively, the drive unit may include a manual drive such a mechanical spring or elastic member that has to be loaded and biased before an injection.

The reservoir may be a container, cartridge (one or two chamber) adapted to contain the drug. The cartridge may be a non-refillable cartridge for single use. The cartridge includes a needle mounting portion where a needle assembly can be releasably attached to. Alternatively, the reservoir may be a prefilled syringe having a needle or cannula that is permanently attached to a first end of a syringe barrel that is sealed at the opposing end by the plunger.

The drive unit preferably includes the device housing comprising an opening to accommodate a proximal portion of the reservoir unit in an attached state. The emitter element is preferably arranged inside the opening and the cap protrudes inside the opening in an attached or mounted state such that the capturing surface is inside the opening. The capturing surface is thus protected against environmental impacts and a reliable transmission of the radiation from the emitter element to cap via capturing surface is provided.

The invention further relates to a method for transmitting an electromagnetic radiation to a cap of an injection device assembly. The method comprising the steps of
a) activating, by a controller, a source generating the electromagnetic radiation and emitting by an emitter element coupled to the source, electromagnetic radiation with a first wavelength;
b) transferring, the emitted radiation via capturing surface to the cap releasably attached to a distal end of the injection device housing;
c) unlocking, by the controller, a locking mechanism of the injection device assembly; and
d) generating, emitting and transferring to the cap an electromagnetic radiation with a second wavelength different to the first wavelength.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a reusable autoinjector comprising a reservoir unit and a drive unit;
- Fig.2: depicts a sectional view of the autoinjector of figure 1 with a light emitter and cap according to the invention;
- Fig.3: depicts an enlarged view a distal portion of the sectional view of figure 2;
- Fig.4: depicts an enlarged view of the emitter according to a second embodiment and
- Fig.5: depicts a plane view of a reservoir unit with a cap according to a third embodiment.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

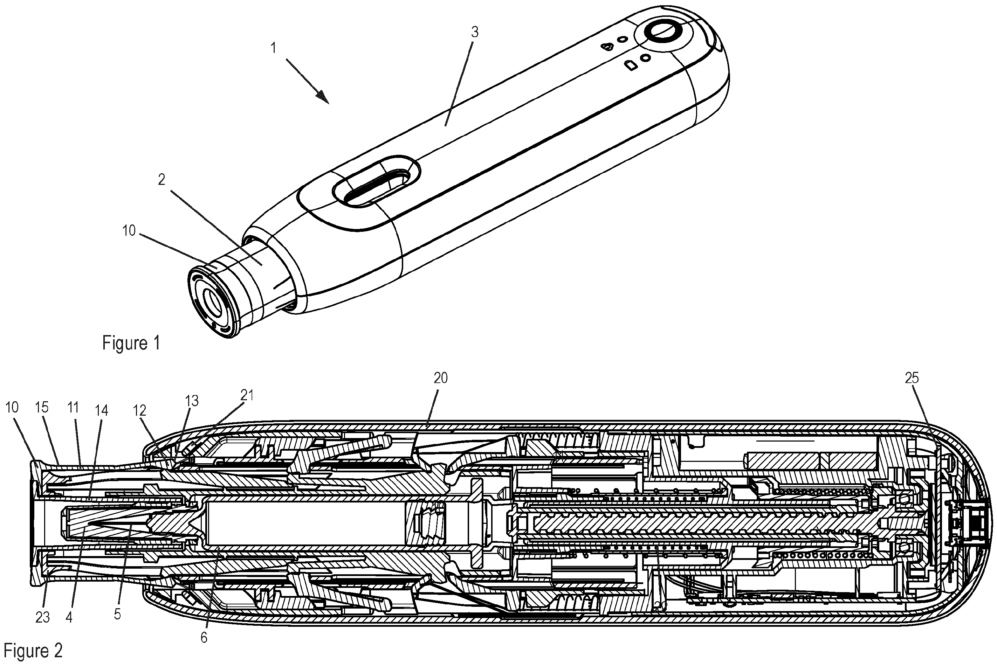

Figure 1 depicts a perspective view of an injection device of a first embodiment according to the invention in form of a semi-reusable autoinjector 1. The autoinjector 1 comprises a disposable reservoir unit 2 and a reusable drive unit 3. As shown in figure 1 the drive unit 3 has an elongated housing defining a longitudinal axis. The drive unit 3 includes an opening or cavity adapted to accommodate a proximal portion of the reservoir unit 2 to attach or couple the reservoir unit 2 to the drive unit 3. The reservoir unit 2 has an elliptical form in the cross section and extends along the longitudinal axis. The reservoir unit holds the reservoir in form of a prefilled syringe with a liquid drug. On a distal end of the reservoir unit a cap 10 is attached in an initial state or shipping state.

The reusable autoinjector with its reservoir unit and drive unit are descripted in detail in the published patent application EP 4275719 A2 (Ypsomed) which is incorporated herein by reference.

Figure 2 depicts a sectional view of the autoinjector with the drive unit 3 and the attached reservoir unit 2 in a shipping state. The cut for the sectional view runs along the longitudinal axis of the autoinjector.

As shown in figure 2 the drive unit 3 comprises in a distal end portion two oppositely arranged radiation emitter in form of LED 21. In a first embodiment the LED 21 generate electromagnetic radiation in form of visible light and emit the light towards the cap 10 which is attached to a distal end of the reservoir unit 2.

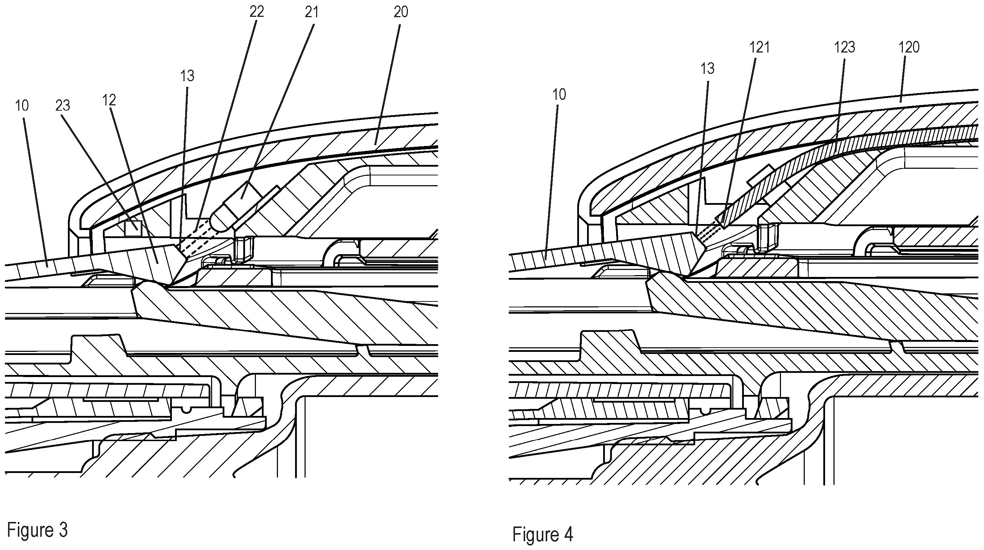

The two LED 21 are located inside a drive unit housing 20 in a distal end portion and are oriented such that the light radiation is directed towards a capturing surface 13 of the cap 10. More precisely, a longitudinal axis of each LED 21 is oriented in an angle of about 135° (or -45°) to the longitudinal axis of the autoinjector 1. That means the light radiation 22 (see figure 3) mainly radiates from the LED 21 in an angle to the longitudinal axis of about 135°.

The LED 21 are electronically connected to an electronic module 25 (figure 2) located in a proximal end portion of the drive unit 3. The LED 21 are supplied by a power source of the electronic module 25.

As shown in figure 2 the cap 10 of the reservoir unit 2 includes a circumferential outer wall 11 forming an opening. At a proximal end 12 the outer wall comprises two oppositely arranged projections adapted to engage a nose of the reservoir unit 2 to form a releasable snap-fit connection such that the cap 10 is releasably hold on the reservoir unit 2. Inside the opening of the outer wall 11 is an inner holding structure 14 in form of a RNS remover sleeve 14 connectable to a rigid needle shield (RNS) 4 of the syringe 6 inside the reservoir unit 2 such that the RNS 4 is removed along with the cap 10 if the cap 10 is released from reservoir unit 2. The RNS remover sleeve 14 is made of an elastomer and is shrunken onto the RNS 4 during assembly of the reservoir unit 2. The outer wall 11 and the RNS remover sleeve 14 are fixedly connected to each other.

The outer wall 11 of the cap 10 is made of translucent plastics with a radiation emitting surface 15 adapted to emit radiation as an indication to a user. The translucent material includes about 10% scattering white pigments. As the RNS remover sleeve 14 is made of elastomer the cap is made by two-component injection molding. The cap 10 with the RNS remover sleeve 14 are descripted in the patent application EP23165348.6 (Ypsomed) in detail which is incorporated herein by reference.

At a proximal end portion 12 of the outer wall 11 the two oppositely arranged capturing surfaces 13 are located. The capturing surfaces 13 are formed as a chamfer or taper. That means the capturing surfaces 13 are angled to the longitudinal axis in an angle of 45° and are positioned opposite the LED 21 when the cap is mounted to the reservoir unit 2 and when the reservoir unit 2 is attached to the drive unit 3. The capturing surfaces 13 are polished or smooth surfaces for an optimal entry of the radiation. The emitting surface 15 is rough or unpolished to provide an optimal emittance of the radiation to the environment.

Figure 3 depicts an enlarged view of the region of the proximal end portion of the cap 10 and the distal end portion with the opening of the drive unit housing 20 with the LED 21. As shown in figure 3 the light radiation 22 depicted as doted lines emitted by the LED 21 hits and enters the capturing surface 13 orthogonal, e.g. in an angle of 90° between the capturing surface 13 and an emission axis or main direction of the light radiation 22.

As mentioned above the outer wall 11 of the cap 10 is made of translucent material. That means if the LED 21 is active and emits light radiation 22 the radiation is transmitted from the LED 21 to the cap 10 via capturing surface 13 and is further transmitted within the cap outer wall 11 and emitted to the environment by a rough or unpolished cap emitting surface 15. In other words, the user can see the light as the outer wall 11 is illuminated.

A controller of the electronic module 25 in the drive unit 3 controls the LED 21. After the reservoir unit 2 has been inserted and completely attached to the drive unit 3 an NFC tag or code on the reservoir unit 2 is read by a reader of the drive unit and the read information is sent to an external server with a database for verification. If the verification (expiry date, type of medication, and/or accordance with a user therapy plan) is positive the controller controls the LED 21 to emit a corresponding signal to the user, e.g. for example the translucent cap is illuminated with a green color. The LED 21 may emit a constant light or may emit light with a not constant and flashing pattern. That can indicate the user that the cap 10 can be removed from the reservoir unit 2 and that the autoinjector 1 is ready for the injection.

In case the verification is negative, e.g. if a wrong reservoir unit is attached or/and the medication is not correct or is expired the controller may control the LED 21 to emit a different light, e.g. the cap may be illuminated with a constant or flashing red color indicating the user that the device is not ready to use. Alternatively, or instead, a red or distinct color may be used to signal the user when an error occurred, for example, if the plunger rod is blocked or the dispensing mechanism has otherwise a malfunction.

The above-mentioned communication to an external device and verification is not mandatory. In an alternative to the above, the controller may activate the LED upon correct insertion of the reservoir unit into the drive unit.

In a further embodiment the autoinjector 1 includes a lock mechanism adapted to selectively lock or unlock the plunger rod or driver to block or allow a dispensing movement. The controller controls the LED to emit a red light if the dispensing mechanism is locked in a shipping state. If the device is unlocked and the drive is idle the controller controls the LED to emit a green light to notify the user that the autoinjector is ready and that the cap can be removed.

Figure 3 further depicts an embodiment with a radiation sensor in form of a photodetector 23 configured to detect light emitted by the cap 10. The photodetector 23 is arranged in the distal portion of the drive unit housing 20 and is electrically connected to the electronic module 25 which supplies the photodetector 23.

The photodetector 23 generates a sensor signal depending on the detected light. When the cap 10 is illuminated by the LED 21 and thus reflects and emits light the photodetector 23 generates a corresponding signal which is different to a signal when no light is detected. Therefore, based on the sensor signal the controller in the electronic module 25 is configured to determine whether the cap 10 is attached to the reservoir unit 2 or if no cap is present. If the controller determines that the cap has been removed it can notify the user and/or prepare the autoinjector for the injection.

Figure 4 depicts a detail of a sectional view of a second embodiment of the present invention. In contrast to the first embodiment the radiation source and the radiation emitter are not the same component. The housing 120 the drive unit includes an optic fibre cable 123 with a first end connected to a light source and a second free end 121 which serves as light emitter. The light source is in the electronic module in the proximal portion of the drive unit housing 120 and the optic fibre cable 123 transmits the light radiation from the source to the emitter in form of the second end 121 of optic fibre cable 123 in a distal end portion of the device housing 120 near the opening as shown in figure 4. The second end 121 of the optic fibre cable 123 is arranged in an angle of about 45 degrees to the longitudinal axis of the autoinjector 1 and a main radiation direction is perpendicular to the capturing surface 13 of the cap as described with respect to the first embodiment above. The cap 10 with its capturing surface 13 is the same as in the first embodiment.

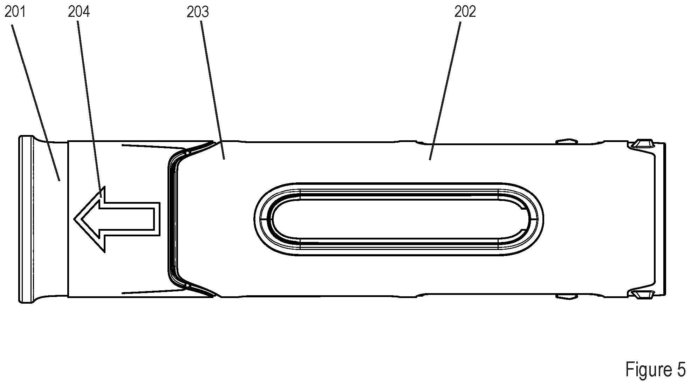

Figure 5 depicts a reservoir unit 202 without the drive unit of the autoinjector of a third embodiment of the present invention. In the third embodiment the radiation emitter emits UV radiation, which is not visible under daylight to the user. The UV emitter may be an emitter diode or alternatively an optic fibre cable as described with respect to the second embodiment.

The reservoir unit 202 as shown in figure 5 includes a main body 203 and a cap 201 releasably attached to the body 203 as described above for the autoinjector 1 in the first and second embodiment. However, the cap 201 includes an arrow sign 204 printed or applied with UV-fluorescent ink onto the surface of the cap 201. The arrow sign 204 is not visible to the user under daylight. When the controller actives the UV emitter the UV radiation is transferred from the emitter to the cap 201 via capturing surface and distributed within the translucent cap 201 (as described with respect to the first and second embodiment). The UV radiation is not visible to the user but upon contact with the UV radiation the arrow sign 204 becomes visible to the user and thus indicates the user that the cap 201 can be removed.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | Autoinjector | 201 | cap |
| 2 | Reservoir unit | 202 | reservoir unit |
| 3 | Drive unit | 203 | body |
| 4 | Rigid needle sleeve | 204 | arrow sign |
| 5 | Needle | | |
| 6 | Syringe | | |
| 10 | Cap | | |
| 11 | outer wall | | |
| 12 | end portion | | |
| 13 | radiation capturing surface | | |
| 14 | elastic sleeve | | |
| 15 | emitting surface | | |
| 20 | housing | | |
| 21 | LED | | |
| 22 | radiation | | |
| 23 | photodetector | | |
| 25 | electronic unit | | |
| 120 | housing | | |
| 121 | second end | | |
| 123 | optic fibre cable | | |

## Claims

1. An injection device assembly for dispensing a liquid drug from a reservoir (6) through an injection needle (5), the injection device assembly comprising
- a device housing (20) for accommodating a dispensing mechanism,
- a radiation emitter element (21) arranged in a distal end portion of the device housing (20) and configured to emit electromagnetic radiation (22),
- a cap (10) releasably attachable to a distal end of the device housing, wherein the cap (10) is attached in a storage state or shipping state and can be removed by the user prior injection,
**characterized in that** the cap (10) comprises a translucent or transparent part with a radiation capturing surface (13) adapted to capture electromagnetic radiation (22) emitted by the emitter element (21) and with a radiation emitting surface (15) adapted to emit, based on the radiation captured, an indication to a user.

2. Injection device assembly according to claim 1, wherein the radiation capturing surface (13) is positioned perpendicular to an emission axis of the electromagnetic radiation (22) such that the radiation (22) can enter the capturing surface (13) in orthogonal orientation.

3. Injection device assembly according to claim 1 or 2, wherein the radiation capturing surface (13) is a smooth or polished surface and wherein an outer surface of the rest of the translucent or transparent part is a rough or unpolished surface with an dispersive effect.

4. Injection device assembly according to any of claims 1 to 3, wherein the radiation emitter element (21) is a LED.

5. Injection device assembly according to any of claims 1 to 4, wherein the radiation emitter is formed by a second end (121) of a fibre optic guide (123) and wherein a first end of the fibre optic guide is optically coupled to a radiation source generating the electromagnetic radiation (22), wherein the radiation source is spatially separated from the radiation emitter element and the electromagnetic radiation is guided by the fibre optic guide (123) from its first end to its second end (121).

6. Injection device assembly according to any of claims 1 to 5, wherein the injection device assembly includes a radiation sensor (23) not arranged along the emission axis, configured to detect electromagnetic radiation emitted by the radiation emitter element (21), transmitted by the translucent or transparent part the cap (10) and wherein the sensor (23) is configured to generate a sensor signal based on the detection and wherein the injection device assembly further includes a controller adapted to determine based on the sensor signal whether or not the cap (10) is attached to the device housing (20).

7. Injection device assembly according to any of claims 1 to 6, wherein the injection device assembly further includes a lock mechanism preventing a dispensing with the dispensing mechanism in a locked state or allowing a dispensing in an unlocked state, wherein in the locked state a radiation in form of visible light of a first colour is emitted and in the unlocked state a light of a second colour different than the first colour is emitted by the radiation emitter element (21).

8. Injection device assembly according to any of claims 1 to 6, wherein the electromagnetic radiation is UV radiation and wherein the cap (201) includes UV-fluorescent printing or ink (204) adjacent to or on the translucent or transparent part and which printing or ink is not visible under daylight but exclusively visible when the emitter element emits UV radiation.

9. Injection device assembly according to claim 8, wherein the UV-fluorescent printing or ink (204) indicates a cap removal sign and wherein the radiation emitter element provides the UV radiation when the injection device assembly is ready for use.

10. Injection device assembly according to any of claims 1 to 9, wherein the translucent or transparent part includes scattering pigments of white colour.

11. Injection device assembly according to any of claims 1 to 10, wherein the cap (10) includes a circumferential outer wall (11) and an inner holding structure (14) adapted to be connected to a rigid needle shield (4), wherein the outer wall (11) comprises the translucent or transparent part and wherein the radiation capturing surface (13) is arranged on a proximal end of the outer wall (11).

12. Injection device assembly according to claim 11, wherein the inner holding structure is an elastic tubular sleeve.

13. An injection device (1) including the injection device assembly according to any of claims 1 to 12, wherein the injection device (1) is a semi-reusable device comprising a disposable reservoir unit (2) adapted to hold the reservoir (6) with the drug and including the cap (10) with the radiation capturing surface (13) and a reusable drive unit (3) including the device housing (20) and the radiation emitter (21).

14. Injection device (1) according to claim 13, wherein the device housing includes an opening to accommodate a proximal portion of the reservoir unit (2) in an attached state, wherein the radiation emitter (21) is arranged next to the opening and wherein the cap (10) protrudes inside the opening such that the radiation capturing surface (13) is inside the opening in the attached state.

15. Method for transmitting an electromagnetic radiation (22) to a cap (10) of an injection device assembly, the method comprising the steps of
• activating, by a controller, a source generating the electromagnetic radiation (22) and emitting by an emitter element (21) coupled to the source, the electromagnetic radiation (22) with a first wavelength;
• transmitting, the emitted radiation (22) to the device cap (10) releasably attached to a distal end of the device housing;
• unlocking, by the controller, a locking mechanism of the injection device assembly; and
• generating, emitting and transmitting to the device cap (10) an electromagnetic radiation with a second wavelength different to the first wavelength.
